# DEMANDE DE BREVET EUROPEEN

(11) **EP 2 733 487 A1**
(43) Date de publication de la demande: **21.05.2014**
(21) Numéro de dépôt: 13190288.4
(22) Date de dépôt: 25.10.2013
(51) Int. Cl.: G01N 33/18, G01N 35/10, G01N 27/62

(54) **Procédé de dosage d'un élément présent en tout ou partie sous la forme de particules en suspension dans un liquide**

(30) Priorité: 16.11.2012 FR 1260918
(71) Demandeur: COMMISSARIAT A L'ENERGIE ATOMIQUE ET AUX ENERGIES ALTERNATIVES, 75015 Paris (FR)
(72) Inventeur: Geertsen, Valérie, 78000 VERSAILLES (FR); Spalla, Olivier, 75019 PARIS (FR); Tabarant, Michel, 91120 PALAISEAU (FR)
(74) Mandataire: Nony

(57) **Abrégé**

La présente invention concerne un procédé de dosage d'un élément présent en tout ou partie sous la forme de particules en suspension dans un liquide, comprenant au moins les étapes consistant en :
(i) disposer d'un échantillon de ladite suspension à analyser, supplémenté avec au moins un agent anti-radicaux libres hydrocarboné ;
(ii) soumettre ledit échantillon (i) à une sonication, réalisée à une température contrôlée pour prévenir tout échauffement de l'échantillon susceptible d'activer des réactions impliquant les particules dudit élément à doser, ces réactions pouvant résultées dans la dégradation des particules sous l'effet de radicaux libres générés par la sonication;
(iii) doser ledit élément par analyse spectrométrique, telle que la spectrométrie de masse, de l'échantillon (ii) ;

l'étape (iii) étant réalisée consécutivement ou simultanément à l'étape (ii) de sonication.

## Description

La présente invention se rapporte à un nouveau procédé de dosage d'un élément présent en tout ou partie sous la forme de particules, notamment de nanoparticules, en suspension dans un liquide.

Le procédé de l'invention trouve une application particulièrement intéressante dans la détermination de la teneur en dioxyde de titane ou en dioxyde de cérium dans une eau naturelle.

Le fort développement des nanotechnologies conduit à la production en quantité de nouveaux nanomatériaux. Pour des raisons évidentes, l'utilisation et/ou le recyclage de produits de consommation courante contenant ces nanomatériaux est susceptible de conduire à leur libération dans les sols et les eaux de surface ([1], [2]). Une voie de transport à grande échelle est certainement celle offerte par les eaux naturelles. Au-delà de l'environnement, une exposition humaine pourrait alors être générée par la contamination des eaux servant à l'alimentation des villes. Or, il a été montré que certains de ces nanomatériaux peuvent présenter une toxicité *in vitro.* La prévention de tout impact potentiel des nanoparticules sur l'environnement et la santé humaine relève donc d'une préoccupation grandissante depuis plusieurs années.

Il apparaît en particulier indispensable de pouvoir procéder à des contrôles réguliers de la teneur en nanoparticules dans les eaux concernées. Or, une des difficultés rencontrées dans ce type de dosage est liée à la coexistence dans ces milieux aqueux des formes solubilisées et non solubilisées pour certains matériaux nanoparticulaires.

Ainsi des études antérieures ont montré qu'il est nécessaire de considérer la solubilité des nanoparticules, notamment lorsqu'elles contiennent des atomes dont la toxicité est avérée (Cadmium, Zinc). En revanche, pour ce qui est du dioxyde de titane, il est extrêmement peu soluble dans l'eau et donc présent pour l'essentiel dans une eau naturelle sous forme particulaire (de taille nanométrique et au-delà).

Or, les nanoparticules de dioxyde de titane sont largement utilisées dans les crèmes solaires, les peintures, les revêtements auto-nettoyants, les cellules solaires, en tant qu'additif alimentaire, et pour des procédés de nettoyage de l'eau par photocatalyse. En outre, plusieurs études ont rapporté que les nanoparticules de dioxyde de titane sont susceptibles de provoquer des réactions inflammatoires chez les cellules de mammifères. Des effets génotoxiques ont également été observés.

Enfin, il n'existe pas aujourd'hui de méthode permettant la mesure directe, reproductible et juste de concentrations faibles (inférieures à 1 ppm) de nanoparticules en suspension.

Il est donc impératif de pouvoir disposer d'une méthode simple et fiable pour déterminer et/ou contrôler la teneur en nanoparticules TiO₂ dans une eau complexe, comme l'est une eau naturelle.

La spectrométrie et la spectroscopie à plasma à couplage inductif (ICPMS et ICPOES) sont des techniques très répandues dans les laboratoires aussi bien industriels que de recherche pour l'analyse quantitative élémentaire ou isotopique. Elles permettent d'analyser des éléments dissous en phase liquide mais aussi des particules transportées dans un flux gazeux (couplage avec l'ablation laser). Il est généralement admis que la source ICP peut ioniser des particules atteignant 3 µm. Il s'agit donc de techniques particulièrement adaptées à la mesure quantitative de nanoparticules comme l'oxyde de titane en suspension.

Peu de publications s'intéressent toutefois à la possibilité d'effectuer des mesures directes en suspension. Gibson *et al.* [3] décrivent notamment les difficultés liées à l'étude de la mesure par ICPMS de concentration en particules de TiO₂ en suspension. Devant l'impossibilité d'obtenir un résultat juste, ils proposent l'utilisation d'une nébulisation par vaporisation (ETV). Dans ce cas, l'échantillon liquide est transformé en échantillon solide afin d'être analysé. Ce type de nébulisation est toutefois difficile à mettre en oeuvre et incompatible avec un laboratoire d'analyse de routine.

Une autre possibilité consiste à solubiliser l'ensemble de la matière (particules et nanoparticules). On peut citer, par exemple, la technique de dosage du titane proposée par Radhamani *et al.* [4], consistant à mettre en solution du TiO₂ solide par fusion phosphate. Toutefois, cette technique nécessite de mettre au point, pour chaque type d'élément, une procédure de solubilisation. Egalement, pour un même élément, la forme chimique de la particule peut entrainer une modification du protocole. Ceci est incompatible avec des échantillons réels contenant des particules naturelles ou industrielles. En outre, ce type de méthode est très long (plusieurs jours en général) car il faut commencer par porter à sec les suspensions liquides (évaporation douce) avant la mise en solution.

Par conséquent, il demeure un besoin de disposer d'une méthode de dosage, fiable et aisée à mettre en oeuvre, d'un élément présent en tout ou partie sous la forme de particules en suspension dans un liquide, et plus particulièrement pour le dosage du titane dans une eau naturelle.

La présente invention vise précisément à répondre à ce besoin.

Plus particulièrement, elle concerne, selon un premier de ses aspects, un procédé de dosage d'un élément présent en tout ou partie sous la forme de particules en suspension dans un liquide, comprenant au moins les étapes consistant en :
(i) disposer d'un échantillon de ladite suspension à analyser, supplémenté avec au moins un agent anti-radicaux libres hydrocarboné ;
(ii) soumettre ledit échantillon (i) à une sonication, dans des conditions non réactives à l'égard des particules dudit élément à doser ; et
(iii) doser ledit élément par analyse spectrométrique de l'échantillon (ii) ;
   l'étape (iii) étant réalisée consécutivement ou simultanément à l'étape (ii) de sonication.

Avantageusement, la sonication est opérée à une température contrôlée pour prévenir tout échauffement de l'échantillon susceptible d'activer des réactions impliquant les particules dudit élément à doser.

On désignera par la suite par « suspension à analyser » ou plus simplement « suspension », la suspension comprenant l'élément que l'on veut doser.

Egalement, on parlera de « l'élément à doser », ou plus simplement « l'élément », encore appelé « analyte », l'élément dont on cherche à déterminer la concentration dans l'échantillon.

Par « particules », on entend désigner à la fois des nanoparticules et des particules de taille supérieure.

L'élément à doser peut être présent sous diverses formes dans la suspension à analyser. Il peut être, par exemple, en partie solubilisé dans la suspension, et en partie sous la forme de nanoparticules et/ou sous la forme de particules de taille supérieure.

Selon un mode de réalisation particulier, le procédé de l'invention est mis en oeuvre pour déterminer la teneur en dioxyde de titane, en particulier dans une eau naturelle telle qu'une eau de rivière. Dans le cadre de ce mode de réalisation, l'élément à doser est l'élément titane.

Selon encore un autre mode de réalisation particulier, le procédé de l'invention est mis en oeuvre pour déterminer la teneur en dioxyde de cérium, en particulier dans une eau naturelle telle qu'une eau de rivière. Dans le cadre de ce mode de réalisation, l'élément à doser est l'élément cérium.

Le procédé de l'invention s'avère particulièrement avantageux à plusieurs titres.

Tout d'abord, comme illustré dans les exemples qui suivent, il permet de déterminer de manière fiable et aisée, la teneur totale en élément, tel que par exemple en titane ou en cérium, présent en tout ou partie sous forme de particules, directement en suspension, sans nécessiter au préalable la mise en solution de cet élément.

Par ailleurs, comme développé par la suite, contrairement aux méthodes de dosage classiques qui requièrent une analyse immédiate après le prélèvement de la suspension, le procédé selon l'invention autorise un report de l'analyse *via* le stockage de l'échantillon, pouvant avantageusement aller au-delà de 24 heures.

Selon un autre de ses aspects, la présente invention concerne une installation, notamment pour la mise en oeuvre du procédé de dosage décrit précédemment, comportant au moins :
- un passeur d'échantillons, et
- une cuve à ultrasons, placée sur le passeur d'échantillons, pour recevoir au moins un contenant rempli avec un échantillon à analyser, lors du prélèvement de l'échantillon par le passeur d'échantillons.

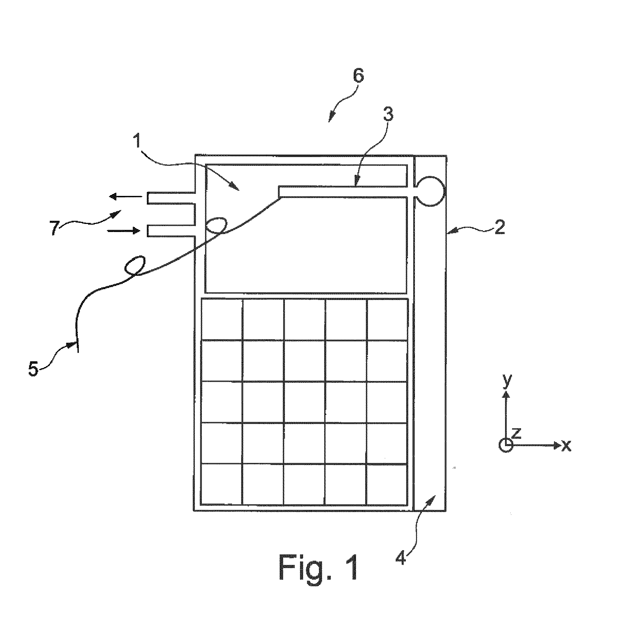

L'invention sera mieux comprise à la lecture de la description détaillée qui va suivre, des exemples de réalisation de l'invention et à l'examen de la figure 1 annexée qui représente, de manière schématique et partielle, une installation selon l'invention.

Il convient de noter que, pour des raisons de clarté, les différents éléments sur la figure 1 sont représentés en échelle libre, les dimensions réelles des différentes parties n'étant pas respectées.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### ECHANTILLON DE L'ETAPE (i)

Une première étape du procédé de l'invention consiste à disposer d'un échantillon de ladite suspension à analyser, supplémenté avec au moins un agent anti-radicaux libres hydrocarboné.

Selon un mode de réalisation particulier, la suspension à analyser est une eau naturelle, par exemple une eau prélevée d'une rivière.

### Agent anti-radicaux libres hydrocarboné

L'agent anti-radicaux libres est plus particulièrement choisi pour sa capacité à neutraliser les radicaux libres susceptibles d'être produits lors de la sonication de l'échantillon.

Les agents anti-radicaux libres sont des composés bien connus de l'homme du métier.

Ils peuvent être plus particulièrement choisis parmi des alcools organiques miscibles à l'eau, notamment l'isopropanol ; la cystéine et ses dérivés comme par exemple la N-acétylcystéine ; l'acide formique, et leurs mélanges.

Selon un mode de réalisation particulier, l'agent anti-radicaux libres mis en oeuvre dans le procédé de l'invention est l'isopropanol.

Le ou lesdits agent(s) anti-radicaux libres peu(ven)t être présent(s) dans l'échantillon (i) en une teneur inférieure ou égale à 20 % en poids, par rapport au poids total dudit échantillon à analyser, en particulier en une teneur allant de 0,001 à 20 % en poids.

### Préparation et stockage de l'échantillon (i)

L'échantillon mis en oeuvre en étape (i) peut être préparé selon un procédé comprenant au moins les étapes consistant en :
(a) ajouter à la suspension à analyser au moins un agent anti-radicaux libres hydrocarboné, en particulier tel que défini précédemment ;
(b) soumettre ledit mélange de l'étape (a) à une sonication, dans des conditions non réactives à l'égard des particules dudit élément à doser ; puis, éventuellement, filtrer ou tamiser ledit échantillon ; et
(c) disposer de l'échantillon (i) ainsi obtenu à l'issue de l'étape (b) à des fins d'analyse ou de stockage.

De préférence, l'échantillon (i) est préparé peu de temps après le prélèvement de la suspension à analyser, en particulier moins de 5 heures après le prélèvement de la suspension pour un volume d'environ 5 L. Il est entendu que plus le volume de suspension prélevé sera grand, plus le temps d'attente entre le prélèvement de la suspension et la préparation de l'échantillon pourra être prolongé.

La sonication de l'étape (b) peut être opérée dans des conditions telles que décrites par la suite pour l'étape (ii) du procédé de dosage selon l'invention.

De manière particulièrement avantageuse, l'échantillon (i) ainsi obtenu en étape (c) peut être conservé avant sa mise en oeuvre dans le procédé de dosage selon l'invention, et son analyse peut ainsi être reportée à un stade ultérieur.

En particulier, l'échantillon ainsi préparé peut être stocké pendant au moins 24 heures, notamment pendant au moins 72 heures.

De préférence, l'échantillon est conservé à l'abri de la lumière. De préférence, il est conservé à une température inférieure ou égale à 15°C, en particulier inférieure ou égale à 10°C.

### ETAPE (ii): SONICATION DE L'ECHANTILLON

Comme précisé précédemment, dans une seconde étape du procédé de dosage selon l'invention, l'échantillon (i) est soumis à une sonication dans des conditions non réactives à l'égard des particules de l'élément à doser.

La sonication peut être réalisée par tout système ultrasonique connu de l'homme du métier, par exemple à l'aide d'un bain à ultrasons, d'une sonde à ultrasons plongeante ou d'une sonde alimentant un réacteur cup-horn.

Selon un mode de réalisation particulier, elle est opérée à l'aide d'un bain à ultrasons.

Bien entendu, le dispositif utilisé peut être choisi au regard de l'élément à doser. Par exemple, dans le cadre du dosage du titane, le système ultrasonique est de préférence de type bain à ultrasons ou sonde alimentant un réacteur cup-horn.

Par l'expression « conditions non réactives », on entend signifier au sens de l'invention que la sonication est opérée dans des conditions propices à l'inertie des particules dudit élément à doser. Autrement dit, l'étape (ii) de sonication selon l'invention ne provoque pas de réactions impliquant les particules de l'élément à doser.

L'homme du métier est à même d'ajuster les conditions opératoires à mettre en oeuvre pour la sonication, de manière à éviter toute réaction parasitaire impliquant les particules d'élément à doser.

Selon l'invention, la sonication en étape (ii) est opérée en contrôlant la température de l'échantillon de manière à prévenir tout échauffement de l'échantillon susceptible d'activer des réactions impliquant les particules dudit élément à doser.

L'homme du métier est à même de mettre en oeuvre des conditions opératoires propices à éviter tout échauffement, même local, de l'échantillon.

Selon un mode de réalisation particulier, le bain de sonication en étape (ii) est maintenu à une température inférieure ou égale à 15°C, en particulier inférieure ou égale à 10°C et plus particulièrement inférieure ou égale à 5°C.

Le bain de sonication peut être par exemple formé d'un mélange eau/glace, ou encore d'un liquide refroidi entre -5°C et 15°C tel que l'éthylène glycol.

Selon un mode de réalisation particulier, le bain est formé d'un mélange eau/glace.

Selon un mode de réalisation particulier, la sonication (ii) est opérée à l'aide d'une cuve à ultrasons remplie d'un mélange eau/glace.

La sonication peut être opérée avec une amplitude des ultrasons variable, notamment en mode pulsation (mode « pulse » en langue anglaise) ou ondulation (mode « sweep » en langue anglaise).

A titre d'exemple, la sonication (ii) peut être opérée en mode pulsation pour alterner des périodes de fonctionnement et des périodes d'arrêt, de manière à éviter un échauffement de l'échantillon, par exemple 1 seconde d'ultrasons/1 seconde pause.

La fréquence du signal ultrasonique en étape (ii) peut être par exemple comprise entre 15 et 100 kHz, en particulier d'environ 40 kHz.

Deux fréquences peuvent par exemple être choisies pour la sonication, une fréquence plus élevée (par exemple de l'ordre de 80 kHz) pour accéder aux surfaces des flacons des échantillons, et une seconde fréquence plus faible (par exemple de l'ordre de 40 kHz) pour pénétrer plus au coeur.

L'homme du métier est à même d'adapter la puissance et les dimensions du système ultrasonique au regard du volume de l'échantillon à analyser.

Egalement, il appartient à l'homme du métier d'ajuster la durée de sonication de manière à casser l'ensemble des agrégats de la suspension et obtenir une suspension de bonne homogénéité.

Notamment, la durée de sonication peut être ajustée selon que le dosage de l'échantillon est effectué après ou pendant la sonication, ou encore en fonction de la durée de stockage.

L'étape (ii) de sonication peut être par exemple opérée pendant une durée allant de 30 secondes à 4 heures.

En particulier, la durée de sonication de l'échantillon avant dosage peut être supérieur pour un échantillon ayant été préalablement stocké pendant un certaine durée, comme décrit précédemment.

A titre d'exemple, l'échantillon peut être soumis aux ultrasons, pendant une durée totale d'environ 45 minutes, avec une fréquence d'environ 40 kHz en mode pulsation 1 seconde d'ultrasons/1 seconde pause.

De préférence, l'échantillon est contenu dans un ou plusieurs contenants, en particulier des flacons.

Les contenants peuvent être en toute matière compatible avec la suspension à analyser. De préférence, ils sont en un matériau ne s'échauffant pas ou peu sous rayonnement ultrasonique, par exemple en verre.

Selon un mode de réalisation particulier, le procédé de l'invention est mis en oeuvre pour le dosage du titane, en particulier dans une eau naturelle, et comprend au moins les étapes consistant en :
(i) disposer d'un échantillon de ladite suspension à analyser, supplémenté avec de l'isopropanol; en particulier à raison de 1 % en volume par rapport au volume total de la suspension ;
(ii) soumettre ledit échantillon (i) à une sonication dans un bain à ultrasons, en mode pulsation (en particulier 1 seconde /1 seconde), le bain étant maintenu à une température inférieure ou égale à 15°C ; et
(iii) doser le titane par analyse de l'échantillon (ii) par spectrométrie à source plasma à couplage inductif ;
   l'étape (iii) étant réalisée consécutivement ou simultanément à l'étape (ii) de sonication, de préférence simultanément.

Selon un autre mode de réalisation particulier, le procédé de l'invention est mis en oeuvre pour le dosage du cérium, en particulier dans une eau naturelle, et comprend au moins les étapes consistant en :
(i) disposer d'un échantillon de ladite suspension à analyser, supplémenté avec de l'isopropanol ; en particulier à raison de 1 % en volume par rapport au volume total de la suspension ;
(ii) soumettre ledit échantillon (i) à une sonication dans un bain à ultrasons, en mode pulsation (en particulier 1 seconde/1 seconde), le bain étant maintenu à une température inférieure ou égale à 0°C ; et
(iii) doser le cérium par analyse de l'échantillon (ii), par spectrométrie à source plasma à couplage inductif ;
   l'étape (iii) étant réalisée consécutivement ou simultanément à l'étape (ii) de sonication, de préférence simultanément.

### ETAPE (iii): DOSAGE PAR ANALYSE SPECTROMETRIQUE

Comme évoqué précédemment, le dosage (iii) dudit élément peut être réalisé consécutivement ou simultanément à la sonication.

Selon une première variante de réalisation, le dosage dudit élément est opéré après l'étape de sonication, de préférence moins de 5 minutes après la fin de la sonication en étape (ii), et plus préférentiellement immédiatement après la fin de la sonication (ii).

Selon une autre variante de réalisation particulièrement préférée, l'échantillon est maintenu sous ultrasons lors du dosage en étape (iii).

L'analyse spectrométrique peut être opérée par toute technique connue de l'homme du métier, par exemple à l'aide d'un spectromètre choisi parmi :
- un spectromètre à source plasma à couplage inductif (ICPS ou en langue anglaise « inductively coupled plasma spectrometer »), et notamment :
   - un spectromètre de masse couplé à une source plasma à couplage inductif (ICPMS) ;
   - un spectromètre optique (ou atomique) couplé à une source plasma à couplage inductif (ICPOES ou ICPAES) ; ou
   - un spectromètre à temps de vol couplé à une source plasma à couplage inductif (ICPTOF) ; ou
- un spectromètre de masse à source électrospray.

Selon un mode de réalisation particulier, le dosage de l'élément en étape (iii) est effectué par spectrométrie à source plasma à couplage inductif, en particulier par spectrométrie de masse ou par spectrométrie d'émission optique.

La méthode de spectrométrie à source plasma induit consiste, d'une manière générale, à introduire en continu l'échantillon dans un nébuliseur où il est vaporisé sous forme d'un brouillard de gouttelettes. Les gouttelettes sont injectées au centre d'un plasma d'argon dans lequel les analytes qu'elles contiennent sont désolvatés, vaporisés, atomisés puis ionisés. Les ions ainsi formés sont introduits à basse pression dans le spectromètre.

Dans une première variante de réalisation, dans le cas de la mise en oeuvre d'un spectromètre de masse à source de plasma induit (ICPMS), les ions sont alors triés selon leur masse/charge et détectés. Pour chaque analyte, les signaux de masse obtenus sont proportionnels à la concentration de l'analyte dans l'échantillon.

Dans une seconde variante de réalisation, dans le cas de la mise en oeuvre d'un spectromètre optique à source de plasma induit (ICPOES), la lumière émise par les ions formés dans le plasma d'argon est analysée dans un spectromètre optique. Pour chaque analyte, les signaux optiques obtenus sont proportionnels à la concentration de l'analyte dans l'échantillon.

Il appartient à l'homme du métier d'apporter les ajustements classiques pour l'analyse de l'échantillon par spectrométrie.

Selon un mode de réalisation particulier, le procédé de l'invention comprend, ultérieurement à la sonication de l'étape (ii) et préalablement au dosage (iii), une étape de filtration ou de tamisage de l'échantillon, lorsque lesdites étapes (ii) et (iii) sont effectuées consécutivement.

L'échantillon peut ainsi être filtré ou tamisé sur filtre ou tamis, après son traitement ultrasonique (ii), et avant l'analyse par spectrométrie.

L'homme du métier est à même de choisir la porosité du filtre ou du tamis, au regard du spectromètre utilisé pour l'analyse, en particulier au regard du diamètre interne du canal du nébuliseur du spectromètre. De préférence, la porosité du filtre ou tamis est choisie de valeur la plus grande possible compatible avec le spectromètre utilisé pour la mesure.

Par exemple, pour un spectromètre possédant un nébuliseur disposant d'un canal de 350 µm de diamètre interne, le filtre ou tamis utilisé peut présenter une taille de pores de 320 µm.

Selon un mode de réalisation particulier, le procédé peut comprendre, préalablement au dosage (iii), une étape d'acidification de l'échantillon à analyser, notamment à une valeur de pH inférieure à 2, en particulier d'environ 1.

Cette étape d'acidification peut intervenir à tout moment avant le dosage, par exemple lors de la préparation de l'échantillon avant sonication, après sonication, le cas échéant avant ou après filtrage.

Le pH de l'échantillon peut être plus particulièrement ajusté par ajout d'un ou plusieurs acides minéraux ou organiques, en particulier choisis parmi l'acide nitrique, l'acide chlorhydrique, l'acide perchlorique, l'acide formique, l'acide sulfurique, l'acide borique, et leurs mélanges.

A titre d'exemple, l'acidification peut être opérée par ajout d'eau régale (mélange de HNO₃ et HCl).

Le ou lesdits acides peu(ven)t être par exemple ajouté(s) à raison de 0,01 à 20 % en volume par rapport au volume total dudit échantillon à analyser.

Pour l'analyse par spectrophotométrie, l'homme du métier est à même de mettre en oeuvre une méthode d'étalonnage connue classiquement utilisée en spectrométrie.

Dans une première variante de réalisation, l'étalonnage est effectué par calibration externe. Elle consiste à mesurer à la fois des échantillons contenant l'analyte dont on veut déterminer la concentration, et des solutions dites « standards » que l'on a préparées à partir de solutions certifiées en concentration. On compare les signaux des échantillons et des solutions standards pour déterminer la teneur de l'analyte dans l'échantillon.

Dans une seconde variante de réalisation, l'étalonnage est effectué à l'aide de la méthode, dite « séquentielle », des ajouts dosés. Elle consiste, dans un premier temps, à analyser un échantillon dont la teneur en analyte est inconnue, puis à ajouter des quantités connues d'analyte. Après chaque ajout, l'échantillon est ré-analysé. Cette technique relativement lourde à mettre en oeuvre (pesée des échantillons et des ajouts à chaque mesure) permet avantageusement de s'affranchir des effets de matrice que l'on peut rencontrer avec la méthode de calibration externe.

Les effets de matrice désignent les différences de comportement d'un analyte donné face à l'instrument de mesure selon les constituants qui l'entourent. De fait, la comparaison du signal donné par une solution inconnue à ceux donnés par des solutions étalons est susceptible d'être faussée.

Il appartient à l'homme du métier d'adapter les conditions de mise en oeuvre de ces méthodes d'étalonnage connues.

Selon un mode de réalisation particulier, l'étalonnage met en oeuvre un étalon interne. L'étalon interne est un élément additionnel mis en oeuvre avec l'une ou l'autre des méthodes d'étalonnage exposées ci-dessus.

Par « étalon interne », on entend un élément, introduit dans l'échantillon avant analyse et dans les standards de calibration, et qui n'était présent avant son ajout, ni dans l'échantillon ni dans les standards. Il est mesuré en même temps que l'élément à doser et sert à corriger les dérives de l'appareil au cours des mesures.

L'homme du métier est à même d'adapter la nature du ou des étalons internes et leurs quantités au regard de la nature de la suspension à analyser.

Par exemple, l'étalon interne pour le dosage de l'élément titane dans une eau naturelle peut être du cobalt. L'étalon interne pour le dosage de l'élément cérium dans une eau naturelle peut être par exemple de l'Europium.

Dans le cas où l'échantillon est stocké, l'étalon interne peut être ajouté avant ou après le stockage de l'échantillon. De préférence, il est ajouté avant le stockage.

Le dosage (iii) peut être avantageusement opéré à l'aide d'un dispositif intégrant un passeur d'échantillons et une cuve à ultrasons, tel que décrit plus particulièrement par la suite.

### INSTALLATION

Comme vu précédemment, la présente invention concerne, selon encore un autre des aspects, une installation adaptée à la mise en oeuvre du procédé de dosage décrit précédemment.

Il sera plus particulièrement fait référence par la suite à la figure 1, qui représente, de manière schématique et partielle, une telle installation.

L'installation (6) selon l'invention comporte plus particulièrement au moins :
- un passeur d'échantillons (2), et
- une cuve à ultrasons (1), placée sur le passeur d'échantillons (2), pour recevoir au moins un contenant rempli avec un échantillon à analyser, lors du prélèvement de l'échantillon par le passeur.

Une telle installation (6) permet avantageusement de maintenir l'échantillon sous ultrasons lors de son dosage.

Les passeurs d'échantillons sont des dispositifs connus de l'homme du métier. Ils servent généralement d'accessoire des spectromètres ICP pour introduire de façon automatique les échantillons à analyser.

L'installation (6) selon l'invention peut comporter un dispositif, non représenté sur la figure 1, de positionnement du ou des contenants remplis par le ou les échantillons.

Ce dispositif peut être fixé de façon amovible dans la cuve, notamment par encliquetage.

Le mot « encliquetage » recouvre toute situation dans laquelle deux pièces distinctes sont assemblées, réversiblement ou irréversiblement, moyennant un certain effort de tension ou compression exercé sur au moins une des pièces à assembler, ledit effort jouant sur l'élasticité desdites pièces.

Il peut s'agir par exemple d'un panier clipsable. Un tel dispositif permet de maintenir, dans des positions définies, les contenants dans le bain.

Les contenants peuvent être des flacons, en particulier en une matière ne s'échauffant pas ou peu sous l'effet des ultrasons, par exemple en verre.

Outre les flacons remplis du ou des échantillons à analyser, l'installation (6) peut également comprendre un ou plusieurs flacons, positionnés en dehors de la cuve à ultrasons, contenant par exemple une solution de calibration qui sera prélevée par le passeur d'échantillons lors de l'analyse, ou une solution de lavage, permettant de rincer le capillaire de prélèvement et le système d'introduction du spectromètre, entre deux prélèvements d'échantillon.

Le passeur d'échantillons selon l'invention comporte, d'une manière classique, un bras (3) amovible selon les trois directions (x), (y) et (z), muni d'un capillaire (5), par exemple en téflon, apte à prélever l'échantillon. Par exemple, le bras du passeur peut se déplacer suivant la direction (y) dans le chemin (4).

Par le déplacement du bras du passeur d'échantillons, il est possible de plonger successivement le capillaire de prélèvement dans les différents contenants positionnés dans la cuve à ultrasons.

Selon une première variante de réalisation, le capillaire de prélèvement du passeur d'échantillons est connecté directement au nébuliseur du dispositif ICP (non représenté en figure 1) par aspiration naturelle. Ce mode a l'avantage de mettre en contact les échantillons uniquement avec la matière du capillaire, en particulier de la matière téflon, ce qui facilite le lavage entre deux prélèvements.

Selon une autre variante de réalisation, le passeur peut être connecté à une pompe péristaltique. Dans ce cas, l'on effectue de préférence des cycles de lavage et séchage des tubes de la pompe après chaque prélèvement d'échantillon, pour éviter les contaminations inter-échantillon.

Selon encore une autre variante de réalisation, le passeur peut être connecté à un dispositif avec vanne de commutation et pompe seringue.

Selon un mode de réalisation particulier, l'installation (6) selon l'invention comporte un système de refroidissement de la cuve à ultrasons, en particulier par circulation (7) d'un liquide remplissant la cuve. Un tel système permet avantageusement de maintenir durant la sonication (ii) la température souhaitée pour le bain et d'éviter, comme vu précédemment, un échauffement de l'échantillon à analyser.

L'installation (6) selon l'invention peut comprendre en outre un système anti-débordement, non représenté en figure 1, pour éviter toute perte du liquide de refroidissement. Il peut s'agir d'un capteur de niveau avec un système d'alarme. Ces systèmes anti-débordement sont bien connus de l'homme du métier et commercialisés.

Le passeur d'échantillon peut être piloté, en particulier par logiciel, de manière à contrôler le positionnement du bras, la durée de lavage, la durée de prélèvement, la hauteur de prélèvement dans le flacon, etc.

Selon un mode de réalisation particulier, l'installation (6) comprend en outre un système de pilotage (non représenté en figure 1) de la cuve à ultrasons (1). La cuve à ultrasons peut être pilotée soit par un module intégré au passeur, soit par logiciel. Le logiciel peut être intégré dans le système d'exploitation de l'instrument de mesure.

Les paramètres de réglage de la cuve à ultrasons peuvent être par exemple la fréquence des ultrasons, la température du liquide de refroidissement, le mode (pulsation, durée fonctionnement/pause, ondulation ...), la durée, etc..

L'installation (6) peut être intégrée dans une enceinte fermée, afin de diminuer les nuisances sonores.

Bien entendu, l'installation (6) selon l'invention peut comprendre d'autres éléments, non représentés en figure 1, présents classiquement dans des passeurs d'échantillons.

L'invention va maintenant être décrite au moyen des exemples suivant donnés bien entendu à titre illustratif et non limitatif de l'invention.

### EXEMPLES

### EXEMPLE 1

### Dosage du TiO₂ dans l'eau de la Seine

### i. Mesure selon le procédé de l'invention

### Préparation de l'échantillon

5 litres d'eau de Seine sont prélevés et transportés au laboratoire.

Deux heures après le prélèvement, 500 mL de suspension sont prélevés et introduits dans une fiole en verre, et 1 % en volume d'isopropanol est ajouté à la suspension.

La suspension est placée dans une cuve à ultrasons remplie d'un mélange eau/glace et soumise aux ultrasons pendant 15 minutes. Le mode pulsation (1s ultrasons/1 s pause) est choisi afin d'éviter tout chauffage de l'échantillon. La fréquence de pulsation est de 37 kHz.

La suspension est filtrée sur filtre de porosité 320 µm (nylon).

100 g de filtrat sont conditionnés dans un erlenmeyer en verre préalablement lavé (vapeur d'acide nitrique pendant 16 heures, puis vapeur d'eau pendant 16 heures, rincés et séchés à l'étuve).

Un étalon interne (cobalt) de concentration connue (1 mL dosé à 1 ppm) est ajouté. L'échantillon ainsi préparé est conservé au réfrigérateur pendant 24 heures.

### Analyse par spectrométrie

L'analyse est réalisée le lendemain.

L'échantillon est sorti du réfrigérateur et acidifié avant analyse par de l'eau régale (2 % en volume).

Avant et tout au long de l'analyse (sauf au cours des opérations d'ajouts et de pesée), l'échantillon est soumis aux ultrasons (mode pulsation, bain eau/glace, fréquence 37 kHz). Le bain est changé régulièrement afin de conserver un mélange eau/glace. La durée totale de sonication de l'échantillon est de 40 minutes.

L'échantillon est analysé par ICPMS par la méthode des ajouts dosés à la masse 50.

### Résultats

La teneur mesurée en titane dans l'échantillon est de 49 µg/kg (ppb). Elle correspond à une teneur de 82 µg/kg de TiO₂.

### ii. Mesure selon la méthode de préférence par fusion phosphate

La méthode de référence consiste à solubiliser l'oxyde de titane afin d'analyser une solution contenant l'élément sous forme totalement dissoute.

Il s'agit d'une adaptation à un four du mode opératoire proposé par Radhamani *et al.* [4], réalisé dans ce cas sur une flamme. Le four dans la méthode de référence est choisi pour améliorer la sécurité du procédé mais ne constitue qu'une adaptation.

La méthode est réalisée dans des creusets en platine, rincés à l'eau et lavés à la vapeur d'eau (laveur pendant 16 heures) et séchés à l'étuve.

### Préparation de l'échantillon

Le plus rapidement possible après prélèvement, une quantité connue de suspension est filtrée sur filtre de porosité la plus fine possible, ici 0,22 µm. Il est conservé dans une boîte de Pétri.

### Analyse

Le filtre est ensuite déposé dans un creuset en platine fermé par un couvercle en platine. Le filtre est minéralisé dans un four entre 25 et 700°C (rampe 1 °C/min).

Le fondant phosphate est préparé en chauffant dans un creuset en platine à 800°C pendant 4 heures et avant un refroidissement rapide, un mélange équimassique (typiquement 8 grammes) de Na₂HPO₄ et NaH₂PPO₄, H₂O. On obtient un solide vitreux.

Le fondant refroidi est ensuite transféré sur le filtre minéralisé. Le mélange filtre-fondant est chauffé avec couvercle à 1000°C pendant 15 minutes (rampe 10°C/min). Après refroidissement, le solide obtenu est mis en solution dans de l'eau à ébullition.

On obtient ainsi une solution mère dont la teneur en titane est mesurée après dilution par ICPOES (raie Ti 334,950 nm), par méthode des ajouts dosés.

### Résultats

La teneur mesurée en titane de l'eau de Seine est de 48 µg/kg (ppb) correspondant à 80 µg/kg de TiO₂.

Les résultats obtenus par les deux protocoles différents, mettant en jeu deux techniques analytiques différentes, sont totalement cohérents. Ceci permet de valider le procédé proposé selon l'invention et montrer qu'il est possible de mesurer des concentrations de moins de 1 ppm.

### EXEMPLE 2

### Dosage du CeO₂ dans l'eau

### Préparation de l'échantillon

On pèse une quantité de CeO₂(HNO₃)_{0,5}(H₂O)₄ (Opal 18, Rhodia) nanoparticulaire que l'on suspend dans 100 mL d'acide nitrique (pH 1,5) afin de préparer une solution mère de concentration en cérium égale à 350 ppm. La solution mère est diluée en milieu nitrique pH 1,5 avec 1 % en volume d'isopropanol afin d'obtenir des échantillons de concentration connue. Un étalon interne (europium) de concentration connue (1 mL dosé à 10 ppm) est ajouté à chacun des échantillons. Les échantillons sont conditionnés dans des erlenmeyers en verre préalablement lavés (vapeur d'acide nitrique pendant 16 heures, puis vapeur d'eau pendant 16 heures, rincés et séchés à l'étuve) et placés au réfrigérateur. Les teneurs exactes en cérium de chacun des échantillons sont calculées à partir des pesées de réactifs et diluants.

### Analyse par spectrométrie

L'analyse est réalisée le lendemain.

Les échantillons sont sortis du réfrigérateur.

Avant et tout au long de l'analyse (sauf au cours des opérations d'ajouts et de pesée), les échantillons sont soumis aux ultrasons (mode pulsation, bain eau/glace, fréquence 37 kHz). Le bain est changé régulièrement afin de conserver un mélange eau/glace. La durée totale de sonication de chaque échantillon est de 35 ou 50 minutes.

Les échantillons sont analysés par ICPMS à la masse 140 par la méthode de calibration externe.

### Résultats

**TABLEAU 1**

| Echantillon | [Ce 140] attendue (théo) ppb | [Ce 140] mesurée (exp) ppb | Ecart (%) (exp-theo)/theo = justesse de la valeur |
|---|---|---|---|
| Ce 50 ppb | 52,2 | 50,8 | - 2,6 % |
| Ce 500 ppb | 469,4 | 452 | - 3,7 % |

Les écarts entre les valeurs attendues et les valeurs mesurées sont dans la gamme d'incertitude de la technique de mesure.

### Références

[1] Gottschalk et al., Environ. Sci. Technol., 2009, 43 (24), pp 9216-9222 ;
[2] Neal et al., Science of the Total Environment, 2011, 409, 1843-1853;
[3] Gibson et al., Spectrochimica Acta Part B-Atomic Spectroscopy, 2006, 61, 753-758;
[4] Radhamani et al., Talanta, 2007, 71, 1932-1938.

## Revendications

1. Procédé de dosage d'un élément présent en tout ou partie sous la forme de particules en suspension dans un liquide, comprenant au moins les étapes consistant en :
(i) disposer d'un échantillon de ladite suspension à analyser, supplémenté avec au moins un agent anti-radicaux libres hydrocarboné ;
(ii) soumettre ledit échantillon (i) à une sonication réalisée à une température contrôlée pour prévenir tout échauffement de l'échantillon susceptible d'activer des réactions impliquant les particules dudit élément à doser ;
(iii) doser ledit élément par analyse spectrométrique de l'échantillon (ii) ;
l'étape (iii) étant réalisée consécutivement ou simultanément à l'étape (ii) de sonication.

2. Procédé selon la revendication 1, pour déterminer la teneur en dioxyde de titane ou en dioxyde de cérium, notamment dans une eau naturelle, ledit élément à doser étant respectivement l'élément titane ou l'élément cérium.

3. Procédé selon la revendication 1 ou 2, dans lequel ledit agent anti-radicaux libres est choisi parmi les alcools organiques miscibles à l'eau, notamment l'isopropanol ; la cystéine et ses dérivés ; l'acide formique ; et leurs mélanges.

4. Procédé selon l'une quelconque des revendications précédentes, dans lequel le ou lesdits agent(s) anti-radicaux libres sont présents en une teneur inférieure ou égale à 20 % en poids, en particulier allant de 0,001 à 20 % en poids, par rapport au poids total dudit échantillon à analyser.

5. Procédé selon l'une quelconque des revendications précédentes, dans lequel le bain de sonication en étape (ii) est maintenu à une température inférieure ou égale à 15°C, en particulier inférieure ou égale à 10°C, et plus particulièrement inférieure ou égale à 5°C.

6. Procédé selon la revendication précédente, ledit bain étant formé d'un mélange eau/glace.

7. Procédé selon l'une quelconque des revendications précédentes, dans lequel l'étape (ii) de sonication est opérée en mode pulsation ou ondulation.

8. Procédé selon l'une quelconque des revendications précédentes, comprenant, ultérieurement à la sonication de l'étape (ii) et préalablement au dosage (iii),une étape de filtration ou de tamisage de l'échantillon, lorsque lesdites étapes (ii) et (iii) sont effectuées consécutivement.

9. Procédé selon l'une quelconque des revendications précédentes, comprenant, préalablement au dosage (iii) de l'élément par spectrométrie, une étape d'acidification de l'échantillon à analyser, notamment à une valeur de pH inférieure à 2.

10. Procédé selon la revendication précédente, dans lequel le pH est ajusté par ajout d'un ou plusieurs acides minéraux ou organiques, en particulier choisis parmi l'acide nitrique, l'acide chlorhydrique, l'acide perchlorique, l'acide formique, l'acide sulfurique, l'acide borique, et plus particulièrement par ajout d'eau régale.

11. Procédé selon l'une quelconque des revendications précédentes, dans lequel le dosage en étape (iii) est opéré par spectrométrie d'émission optique (ICPOES) ou par spectrométrie de masse (ICPMS).

12. Procédé selon l'une quelconque des revendications précédentes, dans lequel ledit échantillon de l'étape (i) est formé selon un procédé comprenant au moins les étapes consistant en :
(a) ajouter à la suspension à analyser au moins un agent anti-radicaux libres hydrocarboné, en particulier tel que défini selon l'une quelconque des revendications 3 et 4;
(b) soumettre ledit mélange de l'étape (a) à une sonication, dans des conditions non réactives à l'égard des particules dudit élément à doser ; puis, éventuellement, filtrer ou tamiser ledit échantillon ; et
(c) disposer de l'échantillon ainsi obtenu à l'issue de l'étape (b) à des fins de stockage avant analyse.

13. Procédé selon la revendication 12, dans lequel l'étape (b) de sonication est opérée dans des conditions telles que définies selon l'une quelconque des revendications 5 à 8.

14. Installation, pour la mise en oeuvre du procédé de dosage tel que défini selon l'une quelconque des revendications 1 à 13, comportant au moins :
- un passeur d'échantillons, et
- une cuve à ultrasons, placée sur le passeur d'échantillons, pour recevoir au moins un contenant rempli avec un échantillon à analyser, lors du prélèvement de l'échantillon par le passeur d'échantillons.

15. Installation selon la revendication précédente, comportant un système de refroidissement de la cuve à ultrasons, notamment par circulation d'un liquide remplissant la cuve.

16. Installation selon la revendication 14 ou 15, comportant un dispositif de positionnement du ou des contenants remplis par le ou les échantillons, ce dispositif étant de préférence fixé de façon amovible dans la cuve, notamment par encliquetage.
